# FASCICULE DE BREVET EUROPEEN

(11) **EP 2 235 218 B1**
(45) Date de publication et mention de la délivrance du brevet: **19.11.2014**
(21) Numéro de dépôt: 09706518.9
(22) Date de dépôt: 23.01.2009
(51) Int. Cl.: C12Q 1/68

(54) **METHODE, PROCEDE, ET KIT DE DIAGNOSTIC, PRONOSTIC DU CANCER COLORECTAL**
METHODE, VERFAHREN UND KIT ZUR DIAGNOSE ODER PROGNOSE VON KOLOREKTALKARZINOM
METHOD, PROCESS, AND KIT FOR DIAGNOSIS OR PROGNOSIS OF COLORECTAL CANCER

(30) Priorité: 31.01.2008 FR 0800543
(43) Date de publication de la demande: 06.10.2010
(73) Titulaire: Assistance Publique Hôpitaux De Paris, 75001 Paris (FR); Université Paris-Est Créteil Val de Marne, 94010 Creteil Cedex (FR)
(72) Inventeur: SOBHANI, Iradj, 94100 Saint Maur (FR); MANSOUR, Hicham, 92350 Le Plessis Robinson (FR)
(74) Mandataire: Wagret, Frédéric
(86) Numéro de dépôt international: PCT/FR2009/050101
(87) Numéro de publication internationale: WO 2009/095596

(56) Documents cités:
- US-A1- 2005 288 226
- DATABASE Geneseq [Online] 21 mars 2000 (2000-03-21), "Neisseria species ORF cloning PCR primer #263." XP002485842 extrait de EBI accession no. GSN:AAZ54878 Database accession no. AAZ54878
- DATABASE Geneseq [Online] 26 août 2004 (2004-08-26), "Human soft tissue sarcoma-upregulated DNA - SEQ ID 4235." XP002485843 extrait de EBI accession no. GSN:ADQ21415 Database accession no. ADQ21415
- NOSHO KATSUHIKO ET AL: "Genetic and epigenetic profiling in early colorectal tumors and prediction of invasive potential in pT1 (early invasive) colorectal cancers" CARCINOGENESIS, IRL PRESS, LONDON, GB, vol. 28, no. 6, 1 juin 2007 (2007-06-01), pages 1364-1370, XP002458487 ISSN: 0143-3334
- URAKAMI SHINJI ET AL: "Wnt antagonist family genes as biomarkers for diagnosis, staging, and prognosis of renal cell carcinoma using tumor and serum DNA." CLINICAL CANCER RESEARCH : AN OFFICIAL JOURNAL OF THE AMERICAN ASSOCIATION FOR CANCER RESEARCH 1 DEC 2006, vol. 12, no. 23, 1 décembre 2006 (2006-12-01), pages 6989-6997, XP002547094 ISSN: 1078-0432
- URAKAMI S ET AL: "COMBINATION ANALYSIS OF HYPERMETHYLATED WNT-ANTAGONIST FAMILY GENES AS A NOVEL EPIGENETIC BIOMARKER PANEL FOR BLADDER CANCER DETECTION" CLINICAL CANCER RESEARCH, THE AMERICAN ASSOCIATION FOR CANCER RESEARCH, US, vol. 12, no. 7, 1 avril 2006 (2006-04-01), pages 2109-2116, XP003015934 ISSN: 1078-0432
- LENHARD ET AL: "Analysis of promoter methylation in stool: A novel method for the detection of colorectal cancer" CLINICAL GASTROENTEROLOGY AND HEPATOLOGY, AMERICAN GASTROENTEROLOGICAL ASSOCIATION, US, vol. 3, no. 2, 1 février 2005 (2005-02-01), pages 142-149, XP005120680 ISSN: 1542-3565
- EBERT ET AL: "Aristaless-like Homeobox-4 Gene Methylation Is a Potential Marker for Colorectal Adenocarcinomas" GASTROENTEROLOGY, ELSEVIER, PHILADELPHIA, PA, vol. 131, no. 5, 11 novembre 2006 (2006-11-11), pages 1418-1430, XP005725528 ISSN: 0016-5085
- KOHONEN-CORISH M R J ET AL: "Promoter methylation of the mutated in colorectal cancer gene is a frequent early event in colorectal cancer" ONCOGENE, BASINGSTOKE, HANTS, GB, vol. 26, no. 30, 1 juin 2007 (2007-06-01), pages 4435-4441, XP002477017 ISSN: 0950-9232 [extrait le 2007-01-29]
- ZOU HONGZHI ET AL: "Highly methylated genes in colorectal neoplasia: implications for screening." CANCER EPIDEMIOLOGY, BIOMARKERS & PREVENTION : A PUBLICATION OF THE AMERICAN ASSOCIATION FOR CANCER RESEARCH, COSPONSORED BY THE AMERICAN SOCIETY OF PREVENTIVE ONCOLOGY DEC 2007, vol. 16, no. 12, décembre 2007 (2007-12), pages 2686-2696, XP002485840 ISSN: 1055-9965
- TANIGUCHI HIROAKI ET AL: "Frequent epigenetic inactivation of Wnt inhibitory factor-1 in human gastrointestinal cancers" ONCOGENE, vol. 24, no. 53, novembre 2005 (2005-11), pages 7946-7952, XP002485841 ISSN: 0950-9232

## Description

La présente invention concerne une méthode, un procédé et un kit de diagnostic/pronostic permettant la mise en évidence d'un cancer colorectal, en vue de son diagnostic, son pronostic ou lors de son suivi thérapeutique.

Plus particulièrement, la présente invention se rapporte aux amorces d'amplification et aux sondes d'hybridation qui peuvent être mises en oeuvre dans ce procédé, ainsi qu'un kit de diagnostic/pronostic du cancer colorectal.

On entend par « diagnostic », la détermination d'une affection d'une personne atteinte par une pathologie donnée ; par « pronostic », le degré de gravité et l'évolution ultérieure d'une pathologie et par «thérapeutique », un traitement à visée curative proposée au patient.

Le cancer colorectal représente le troisième cancer chez l'homme, la deuxième cause de décès en France et son incidence ne cesse d'augmenter d'année en année.

Son coût pour la société dépend du stade du diagnostic et du pronostic de la maladie. Aux stades très précoces ou immédiatement précancéreux, cette maladie est curable à un coup peu élevé et sa mortalité est nulle. Aux stades plus avancés, son coût économique et son taux de mortalité sont très élevés.

Les facteurs de risque sont en partie dus à notre mode de vie et notre alimentation mais également à notre patrimoine génétique. Généralement les cancers colorectaux héréditaires sont caractérisés soit par une anomalie du gène APC soit par le syndrome de Lynch qui se traduit par des anomalies de gènes codant pour les protéines de réparation de l'ADN. Dans les deux cas, il s'agit soit d'une ou plusieurs mutations géniques soit d'une ou plusieurs anomalies de méthylation de ces gènes ou des gènes régulateurs de ceux-ci.

Au plan histologique, la muqueuse tapissant le colon et le rectum se transforme en crypte aberrante et polype adénomateux jusqu'à devenir une tumeur invasive. Un saignement occulte, détectable dans les selles, peut être le témoin de la tumeur dès lors le polype adénomateux devient visible et aux stades plus avancés. Ces saignements sont intermittents.

Toutefois, au plan génique, il est connu que l'élément initiateur de la carcinogénèse colorectale est, dès le stade de crypte aberrante, l'inactivation du gène APC, dont la protéine joue un rôle relatif au contrôle de la prolifération, de l'apoptose et de la migration des cellules épithéliales intestinales. Cette inactivation provoque l'activation de certaines voies de signalisation, telles que celle de la WNT/Béta-caténine et la formation d'adénomes précoces. Les étapes suivantes de la carcinogenèse colorectale nécessitent le cumul d'autres anomalies géniques telles que les mutations du gène K-RAS, l'inactivation du gène p53 ou encore l'inactivation de gènes impliqués dans le processus de mésappariements de l'ADN (gène MMR).

La réduction de la mortalité due à ce cancer nécessite un diagnostic de la maladie aux stades très précoces. Un dépistage des sujets asymptomatiques âgés de plus de 50 ans est la meilleure action de santé publique dans ce domaine. Actuellement, le dépistage se réalise par un test biologique de recherche de sang occulte dans les selles de type biochimique («Gaïac», «Magstream» ou «Hemoccult») ou immunologique «Magstream» ; «Eiken») suivi d'une coloscopie systématique si ceux-ci sont positifs, afin de détecter les tumeurs et/ou les retirer.

La limite de ces tests est leurs faibles sensibilités ou spécificités. En effet, le test Hemocult est positif dans 13% à 50% des cas avec tumeur colique ou rectale, en même temps près de 50% des individus ayant un test Hemcoult positif auront une coloscopie normale. Le test immunologique a amélioré le taux de sensibilité du fait de son caractère quantitatif dans la recherche de sang fécal mais la spécificité du test risque de diminuer si le seuil de positivité du test est fixé très bas générant ainsi des coloscopies inutiles avec un impact non négligeable sur le ratio coût/efficacité.

Ainsi des tests alternatifs sont nécessaires. Des cellules épithéliales coliques incluent des cellules d'origine tumorale exfoliées quotidiennement dans les selles. Malgré la faible quantité d'ADN humain fécal, les anomalies géniques d'origine tumorale deviennent potentiellement des marqueurs d'intérêt dans un test diagnostic.

Un test commercialisé basé sur la détection de mutation ponctuelle de l'ADN fécal est disponible. Il présente une sensibilité supérieure aux autres tests biologiques (recherche de sang occulte dans les selles) mais se révèle être très onéreux.

Dans la demande de brevet internationale n° WO2005/112988, il est explicité des compositions, des procédés et des kits pour diagnostiquer et traiter des cancers, dans lesquels, le facteur inhibiteur de Wnt ((Wnt Inhibitory Factor1- WIF1) est sous-exprimé. Il est prévu des compositions pour inhiber la prolifération de cellules cancéreuses, pour induire l'apoptose dans une cellule cancéreuse, par inhibition de la signalisation Wnt dans une cellule cancéreuse et pour le traitement d'une maladie associée à la sous expression de WIF1.

Les tests actuels permettent de détecter avec plus ou moins de sensibilité et de spécificité des mutations d'ADN ou des conséquences des mutations (sous expression d'une protéine) ou la présence de sang dans les selles.

Aujourd'hui, le problème est de créer un test de dépistage et/ou de pronostic permettant la découverte et/ou le diagnostic de cancer en phase précoce ayant une bonne spécificité et sensibilité tout en restant abordable en termes de prix.

On connait de la publication « CARCINOGENESIS, vol. 28, n°6, 1 juin 2007, pages 1364-1370 » un procédé de pronostic du cancer colorectal consistant à prélever du matériel biologique, à partir duquel est mesuré le niveau de méthylation de l'ADN en utilisant notamment des sondes de détection de six gènes dont le gène WIF-1. Toutefois, ce procédé nécessite une méthode invasive.

Il est par ailleurs connu de la publication « CLINICAL CANCER RESEARCH, 1 DEC 2006, vol.12, n°23, 1 décembre 2006, pages 6989-6997 » que le sérum peut être utilisé en tant que source de détection de la présence de méthylations dans le DNA, dont le gène WIF-1 parmi d'autres, pour détecter et connaître le degré d'avancement du cancer du rein chez l'individu.

La publication« CLINICAL CANCER RESEARCH, US, vol.12, n°7, 1 avril 2006, pages 2109-2116 » décrit une corrélation entre la présence de méthylation dans le gène WIF-1 dans un prélèvement d'urine et le cancer de la vessie.

Enfin, la publication préconise de détecter la présence de méthylations dans le DNA de selles pour détecter le cancer colorectal.

La présente invention pallie les inconvénients de l'art antérieur en proposant une nouvelle méthode, un procédé et un kit de diagnostic/pronostic permettant la mise en évidence d'un cancer colorectal, en vue du diagnostic, du pronostic ou du suivi thérapeutique.

Le procédé selon l'invention permet notamment de déterminer la présence ou non d'un cancer colorectal, d'évaluer sa gravité, de déterminer le traitement le plus adapté ainsi que de suivi des patients traités.

La présente invention a pour but de remédier aux inconvénients précédemment évoqués et consiste pour cela en un procédé ou méthode selon la revendication 1 ou 2, en son utilisation selon la revendication 8 et en une utilisation d'un kit selon la revendication 9 ou 10.

Avantageusement, les étapes b) et c) sont réalisées en même temps et l'étape b) est une PCR conventionnelle ou quantitative.

La sonde de détection selon l'invention peut comporter un marqueur.

Plus particulièrement, ladite paire d'amorces comprend au moins une amorce d'amplification comprenant au moins 15 motifs nucléotidiques d'une séquence nucléotidique choisie parmi les SEQ ID n° 1 à 10 et/ou ladite sonde de détection comprend au moins 15 motifs nucléotidiques d'une séquence nucléotidique choisie parmi SEQ ID n° 1 à 10.

Plus précisément, la présente invention concerne également une amorce d'amplification pour le diagnostic/pronostic et le suivi du cancer colorectal, pour l'amplification d'une séquence cible comprise dans le gène WIF1, comprenant au moins 15 motifs nucléotidiques d'une séquence nucléotidique choisie parmi les SEQ ID n°1 à 10.

De même, la présente invention se rapporte aussi à une sonde de détection pour le diagnostic/pronostic et le suivi du cancer colorectal, destinée à la détection de méthylations d'une séquence cible comprise dans le gène WIF1, comprenant au moins 15 motifs nucléotidiques d'une séquence nucléotidique choisie parmi SEQ ID n° 1 à 10.

De manière avantageuse, la présente invention concerne l'utilisation de ladite amorce d'amplification et/ou de ladite sonde de détection pour le diagnostic/pronostic et le suivi du cancer colorectal.

L'invention sera bien comprise à la lumière de la description qui suit, se rapportant à des exemples illustratifs, et en aucun cas exhaustifs, de la présente invention, en référence aux dessins, ci-joints, dans lesquels :
- la figure 1 est une représentation d'un gel d'agarose des séquences isolées à partir de l'ADN tissulaire de deux patients (n° 5 cancer colique, n°31 cancer rectal d'une collection biologique);
- la figure 2 est une représentation chromatographique de séquençage de l'amplicon du gène WIF1 qui met également en évidence les sites de méthylation CG ;
- les figures 3 et 4 sont des représentations graphiques illustrant le niveau de méthylation retrouvées dans les urines et le sérum des individus (normaux ou avec tumeur colorectale).

L'inactivation du gène APC engendre l'activation des voies de signalisation des protéines telles que la Wnt et la formation d'adénomes précoces. Il est connu que la voie WIF 1 est l'un des gènes impliqués dans l'inhibition de la voie de signalisation Wnt.

La présente invention propose une méthode, un procédé, un test et un kit de diagnostic/pronostic simplifiés, permettant la mise en évidence d'un cancer colorectal, en vue du diagnostic, du pronostic ou du suivi thérapeutique, basés sur la mise en évidence d'anomalies fonctionnelles de l'ADN, telle que les méthylations.

On sait que l'ADN altéré résulte toujours d'une origine tumorale et qu'il est libéré dans la lumière colique, la circulation sanguine ou les voies urinaires. La recherche d'anomalie, comme par exemple l'ADN méthylé permet de détecter un plus grand nombre de tumeurs, dont celles colorectales.

De plus, il a été démontré que certains phénotypes d'anomalies épigéniques présentant des îlots CpG méthylés (zones où les paires de nucléotides C et G sont très fréquentes) étaient présents dans certains cancers colorectaux. En effet, il a été décrit que l'expression silencieuse de WIF1 est due à un promoteur hyperméthylé.

Préalablement, une récolte d'un « échantillon biologique » est opérée chez un individu susceptible d'être atteint par un cancer ou en quête de dépistage/diagnostic/suivi. Cet échantillon, contenant des cellules circulantes et du matériel nucléique, peut être son sang, ses selles, sa salive, son urine, sa bile, son sérum, son plasma etc.

Le « matériel nucléique », composé de séquences d'acides désoxyribonucléiques (ADN) ou ribonucléiques (ARN), ainsi prélevé chez ledit individu via ledit échantillon biologique comprend une séquence cible.

Une « séquence cible », est une séquence nucléotidique spécifique d'un gène cible qu'il soit simple ou double brin, tel que le WIF1.

Par exemple, voici la séquence du gène WIF1 :

Une fois modifié par du bisulfite de sodium, la séquence d'intérêt ou cible du gène WIF1 complètement méthylé est :

Une « séquence nucléotidique » est un enchaînement de motifs nucléotidiques, c'est-à-dire une séquence d'acides nucléiques ou de polynucléotides ou de fragments de ces derniers.

On entend par « motif nucléotidique » un nucléotide naturel d'acide nucléique ou un nucléotide modifié (base, phosphate, sucre).

Les structures et les modifications de ces enchaînements sont soit naturelles, soit résultantes d'une recombinaison génétique ou d'une synthèse chimique.

Le procédé selon l'invention se rapporte à de nouvelles séquences nucléotidiques d'une séquence cible, telle celle du WIF1, qui peuvent être utilisées en tant qu'amorces d'amplification ou de sondes d'hybridation afin de détecter et/ou pronostiquer et/ou suivre un individu atteint d'un cancer colorectal.

Plus particulièrement, les inventeurs ont découvert que lesdites séquences nucléotidiques cibles présentaient des anomalies fonctionnelles telles que les méthylations.

Ainsi la présente propose une nouvelle méthode, un procédé et un kit de diagnostic/pronostic permettant la mise en évidence d'un cancer colorectal, en vue du diagnostic et/ou du pronostic et/ou du suivi thérapeutique, en recherchant une hyperméthylation du gène de WIF1 ou de son promoteur et en quantifiant cette anomalie.

De manière plus précise, ladite séquence nucléotidique selon l'invention comprenant au moins 15 motifs nucléotidiques d'une séquence choisie parmi les SEQ ID n°1 à n°10 est très pertinente :
- en tant qu'amorce d'amplification pour amplifier les séquences cibles, tel que le gène WIF1,
- en tant que sonde d'hybridation pour une hybridation spécifique sur des séquences cibles, tels que le gène WIF1.

### L'étape a) consiste en une extraction du matériel nucléique d'un échantillon biologique.

La mise en évidence de la présence d'ADN tumoral à partir d'effluent ou d'échantillon biologique nécessite l'extraction de l'ADN total. Cette extraction est réalisée par tout protocole d'extraction d'acides nucléiques d'échantillon biologique de type connu en soi.

Par exemple, cette extraction peut être effectuée par une méthode de lyse des cellules issues de l'échantillon biologique, telle que par exemple chimique, physique, thermique, etc.

Avantageusement, cette étape peut être suivie d'une purification (centrifugation ou toute autre méthode de purification de type connu en soi) consistant à séparer et à concentrer les acides nucléiques des autres constituants.

Les cellules qui échappent à l'apoptose, comme les cellules tumorales, possèdent des fragments d'ADN plus long que ceux de cellules normales. L'ADN long peut donc servir de premier marqueur de tumeur colorectale.

Par exemple, l'échantillon biologique peut être des selles (5 à 10g) disposées dans une solution tampon favorisant l'extraction des acides nucléiques (ADN, ARN), par l'intermédiaire du kit « EXACT » ou « QIAGEN » ou tout autre procédé. Les acides nucléiques obtenus peuvent être suspendus dans un tampon Tris enrichie en EDTA.

Bien entendu, l'homme de l'art sait adapter l'extraction, la purification et la conservation des acides nucléiques en fonction des échantillons biologiques dont ils sont issus.

### L'étape a bis) est la modification de l'ADN extrait.

En vue de procéder à la détection des anomalies fonctionnelles de la séquence cible, il est nécessaire de modifier l'ADN recueilli par tout type de méthode de modification de type connu en soi.

La méthylation d'un gène ou de son promoteur influence son expression et peut entraîner son inactivation fonctionnelle, par exemple.

Les présents inventeurs ont recherché parmi les marqueurs principaux méthylés, le seuil de normalité, puisque le nombre de gènes méthylés et leur niveau de méthylation augmentent avec l'âge.

A titre illustratif, l'ADN recueilli peut être purifié à partir de lymphocytes T, en utilisant le kit « Qiagen » et être artificiellement méthylé par de la méthyltransférase ou toute autre méthode. Ensuite l'ADN ainsi méthylé est modifié, par du bisulfite de sodium avant sa purification.

Le bisulfite de sodium transforme la cytosine non méthylée en uracile alors que la cytosine méthylée reste intacte.

Après cette étape on peut amplifier et quantifier la forme méthylée du gène WIF1.

### Les étapes b) et c) sont : l'utilisation d'amorces d'amplification afin de générer des amplicons d'au moins une séquence cible du matériel nucléique.

Selon la présente invention, une « amorce d'amplification » est une séquence nucléique comprenant 10 à 100 motifs nucléotidiques, préférentiellement de 40 à 80 paires de bases d'au moins une séquence cible de matériel génétique.

Préférentiellement, ladite amorce comprend au moins 15 motifs nucléotidiques d'une séquence choisie parmi :
- une séquence de SEQ ID n°1 à 10 ;
- une séquence homologue de SEQ ID n°1 à 10 complémentaire ou suffisamment complémentaire ; ou présentant une homologie suffisante pour s'hybrider à SEQ ID n°1 à 10 ou à leurs séquences complémentaires ;
- une séquence comprenant une séquence de SEQ ID n°1 à 10, qui aurait la même fonction que l'amorce d'amplification.

Une séquence trop petite améliore le rendement mais se révèle moins spécifique et, à contrario, une séquence trop importante diminue le rendement tout en augmentant la spécificité de la séquence cible.

Plus précisément l'utilisation d'une paire d'amorces permet de réaliser un processus d'amplification par une polymérisation enzymatique (technique de réplication ciblée in vitro dite « PCR », Polymerase Chain Reaction), permettant d'obtenir à partir d'un échantillon d'importantes quantités d'un fragment d'ADN spécifique et de longueur définie.

Ces techniques sont bien connues de l'homme de l'art.

On entend par « hybridation », un processus par lequel deux séquences nucléiques, comme par exemple, une amorce et une séquence cible se lient dans des conditions spécifiques et bien connues de l'homme du métier.

Les séquences d'amorces utilisées peuvent étre :
- WIF1 sens : TATACGTTTATTTCGCGGGC (SEQ ID N°4)
- WIF1 anti sens : ACGACGACCCGACCTCCGCCC (SEQ ID N°5) ou encore :
- WIF1 sens CGTATTTCGTTCGCGTTAGTTTTTTTC (SEQ ID N°6)
- WIF anti sens CTCTCCTCCTACGACGACCCG (SEQ ID N°7)

De manière alternative, les séquences d'amorces et/ou des sondes comprenant comprennent au moins 15 motifs nucléotidiques d'une séquence nucléotidique choisie parmi :
(SEQ ID N°1)
(SEQ ID N°2)
(SEQ ID N° 3)

Préférentiellement, les séquences des amorces et des sondes utilisées sont :
- WIF1 sens : TTTGACGGCGTTAGGTTGC (SEQ ID N°8)
- WIF1 anti sens ACTACTCAAAACCTCCTCGCTACC (SEQ ID N°9)
- la sonde WIF1 : CGGTACGAGGAGTTTT (SEQ ID N°10)

Par exemple, les amorces de méthylation suscitées sont amplifiées par PCR classique ou quantitative sur la matrice lymphocytaire artificiellement méthylée par l'enzyme S-méthyltransférase.

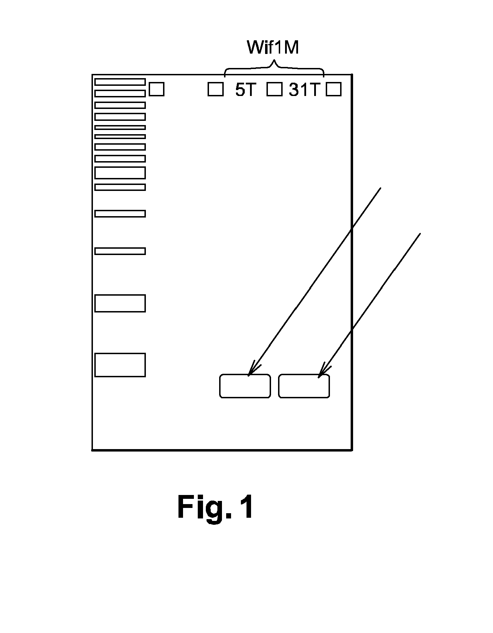

La figure 1 est une représentation d'un gel d'agarose des séquences isolées à partir de l'ADN tissulaire de deux patients (n° 5 cancer colique, n° 31 cancer rectal d'une collection biologique).

Comme illustré à la figure 1, les amplicons peuvent être ensuite analysés sur gel d'agarose pour une électrophorèse afin de vérifier que leurs tailles correspondent bien à la taille attendue des amorces WIF1.

Ainsi on peut visualiser les amplicons issus de la PCR dans la fourchette de poids attendue et correspondant aux 69 paires de bases pour WIF1 comme indiqué sur la figure 1 pour un malade atteint d'une tumeur colique (n°T5) et un autre malade atteint d'une tumeur rectale (n° T31).

Lors de l'étape de la détection des acides nucléiques cibles, il peut être prévu d'utiliser une sonde de détection spécifique.

Bien entendu, cette étape de détection directe ou indirecte, peut être réalisée par tout autre mode de détection de type connu en soi.

Une « sonde d'hybridation » est une séquence nucléique de 10 à 100 motifs nucléotidiques ayant une spécificité d'hybridation dans des conditions spécifiques et déterminées afin qu'elle cette s'hybride avec la séquence nucléique cible.

De manière avantageuse, la sonde d'hybridation est une sonde de détection. En effet, la sonde d'hybridation peut comprendre un marqueur permettant sa détection. Ledit « marqueur » est un traceur capable de repérer, marquer ou distinguer ce qui est reconnaissable du fait de sa propriété physique ou chimique (radioactivité, fluorescence, masse, couleur, luminescence et autres... via des détections optique, électrique, etc.) en très faible quantité.

Les amorces fonctionnelles sont analysées avec un fluorochrome ou un autre fluorescent ou « quencher » qui se lie au produit d'amplification (ADN double brin).

Ainsi on peut détecter une réaction d'hybridation entre une sonde de détection et la séquence cible.

De manière alternative, il peut être prévu que la sonde de détection soit une sonde de détection « molecular beacon ».

La sonde d'hybridation peut également être une sonde de capture, où ladite sonde est immobilisée sur un support solide par tout moyen approprié (de type connu en soi). On détectera ensuite la réaction d'hybridation entre la sonde de capture et la séquence cible.

Pour la détection de la réaction d'hybridation, on peut également utiliser des séquences cibles marquées, directement ou indirectement de la séquence cible.

De manière alternative, l'étape d'hybridation peut être une étape de marquage et/ou de clivage de la séquence cible.

Afin de s'affranchir des dimères d'amorces qui se constituent spontanément, il peut être prévu de lier aux amorces sens et antisens une sonde taqman spécifique construite à l'aide de logiciels bien connus.

Les inventeurs de la présente invention, en vue de corriger toute variabilité d'efficacité enzymatique possible, ont normalisé l'expression du gène cible par la détermination d'un gène de ménage dont l'expression est obligatoire et courante chez tous les individus, par l'intermédiaire d'un ratio.

De manière alternative, l'amplification est réalisée en même temps que l'étape de la détection.

Alternativement, l'amplification est une PCR quantitative permettant de quantifier directement les séquences cibles souhaitées.

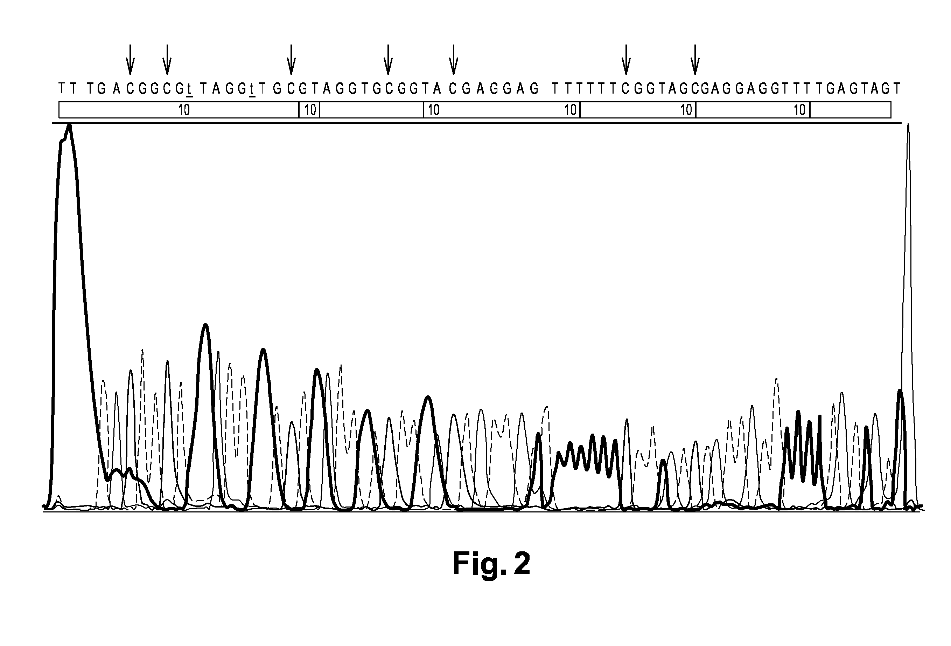

La figure 2 est une représentation chromatographique de séquençage de l'amplicon du gène WIF1 qui met également en évidence les sites de méthylation CG (flèche en haut de la figure) de deux tumeurs de deux patients différents après traitement au bisulfite de sodium.

Les flèches indiquent les cytosines méthylées reconnues par les séquences dinucléotidiques CpG, couvertes par les amorces WIF1.

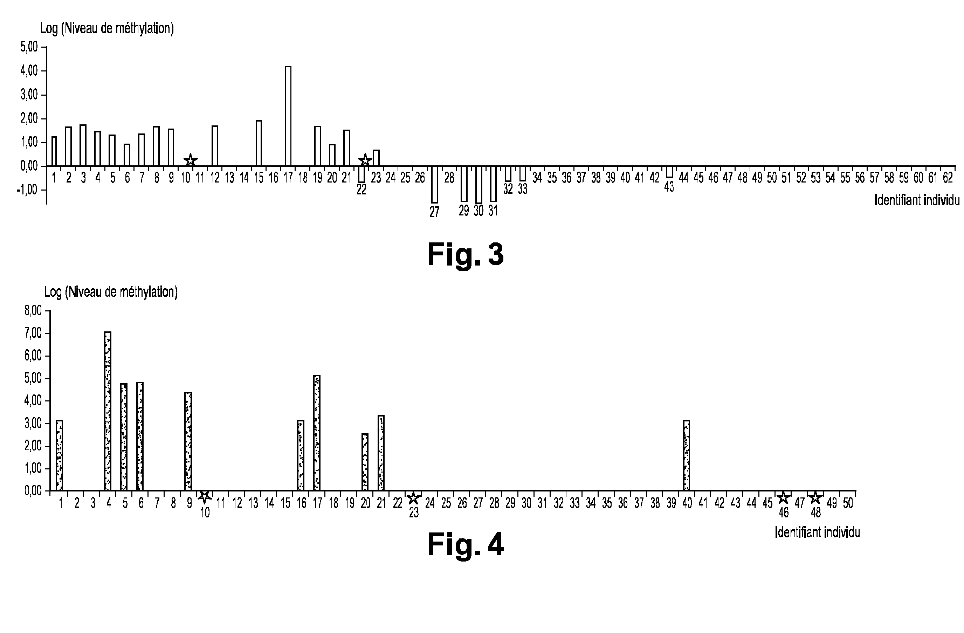

Les figures 3 et 4 sont des représentations graphiques illustrant les niveaux de méthylation retrouvées dans les urines et le sérum des individus normaux et atteints de cancer colorectal.

Le niveau de méthylation est indiqué par la hauteur des barres et les étoiles indiquent une méthylation anormale mais non quantifiable par ce procédé ou tout autre procédé similaire.

Les résultats explicités ci-après sont illustratifs d'expériences de comparaison réalisées et en aucun cas limitatifs.

**Tableau 1: Comparaison des échantillons prélevés chez des individus normaux et atteints de tumeurs plus ou moins développées***

| Diagnostic | Cancer | Adénomateux | Normal | Total |
|---|---|---|---|---|
| Nb. individus | 109 | 42 | 121 | 272 |
| Tissus | 10 | 0 | 10 | 20 |
| Urine | 41 | 7 | 14 | 62 |
| Sérum | 41 | 7 | 14 | 62 |
| Selles | 68 | 35 | 105 | 208 |
| Test total | 160 | 51 | 143 | 352 |

| | | | | |
|---|---|---|---|---|
| * selon les fichiers de la colonoscopie et pathologiques | | | | |

**Tableau 2: Caractéristiques des marqueurs après l'extraction de l'ADN du tissu ou des effluents biologiques**

| Marqueur ADN | Tissus | Selles | Urine | Sérum |
|---|---|---|---|---|
| Muté | | | | |
| Kras12tgt | ND | 208 | ND | ND |
| Kras12ggc | ND | 208 | | |

| Methylé | | | | |
|---|---|---|---|---|
| Alx4 | 10 | 208 | 62 | 62 |
| Vimentine | 10 | 208 | 62 | 62 |
| WIF1 | 10 | 208 | 62 | 62 |

**Tableau 3: Comparaison des méthylation dans le sérum et les urine de WIF1, ALX4 and Vimentine chez les mêmes individus.**

| | **Serum** | | **Urine** | | **Serum &Urine** | |
|---|---|---|---|---|---|---|
| **Genes** | **sensibilité** | **spécificité** | **sensibilité** | **spécificité** | **sensibilité** | **spécificité** |
| **Wif1** | 29.16%(14/48 | 100%(0/14) | 52.08%(25/48) | 92.85%(1/14) | 60.41%(29/48) | 92.85%(1/14) |
| **ALX4** | 14.58%(7/48) | 100%(0/14) | 22.91%(11/48) | 100%(0/14) | 35.41%(17/48) | 100%(0/14) |
| **Vim** | 8.33%(4/48) | 100%(0/14) | [4.16%(2/48) | 100%(0/14) | 10.41%(5/48) | 100%(0/14) |

Comme illustrés sur les tableaux 1 à 3, les échantillons biologiques ont été prélevés sur 272 individus. L'ADN a été extrait à partir de tissus (10 normaux et 10 tumoraux), et les urines, le sérum, les selles et soumis à l'extraction d'ADN, la modification et PCR de méthylation pour identifier le gène cible WIF1, ALx4 et Vimentine. Deux mutations Kras ont été détectées dans les selles comme un contrôle fécal de dosage.

Le marqueur de méthylation de WIF1 a été le plus sensible dans l'urine et le sérum avec une sensibilité de 52,08% et 29,16% respectivement.

La combinaison de ces deux milieux (urine et de sérum) a amélioré la sensibilité de l'épreuve pour tous les gènes marqueurs de dépistage: WIF1 60,41 %, ALX4 35,41 % et Vimentine 10,41 % sans perte de spécificité.

La sensibilité et la spécificité des épreuves combinées (mutations Kras et sur la méthylation) étaient de 23% et 97% respectivement dans les selles.

Les valeurs ont varié comme suit pour la sensibilité et la spécificité des gènes évaluées: Kras1 [9,61% (10/104), 97,16% (3 / 106)], Kras2 [6,73% (7 / 104) et 99,05% (1 / 106) ], [Wif1 9,61% (10/104), 99,05% (1 / 106)], [ALX4 8,65% (9 / 104) et 99,05% (1 / 106)] et [Vim 0,96% (1 / 104); 100% 17 (0 / 106)]. Comme seul test de méthylation, la spécificité et de WIF1 est de 97% pour la détection des tumeurs colorectales.

Bien que combiné avec d'autres marqueurs moléculaires, sa sensibilité est améliorée. Une analyse systématique de la méthylation WIF1 comme seul marqueur dans le sérum et/ou l'urine (voire des selles) de la même personne pourrait offrir une méthode non-invasive pour le dépistage du cancer colorectal.

Les résultats obtenus en comparaison avec d'autres marqueurs démontrent que la sensibilité et la spécificité de cette méthode sont considérablement supérieures. En effet, la sensibilité globale du test de méthylation WIF1 a été évaluée à 58% et sa spécificité à 100%.

Bien entendu, la méthode et le test selon l'invention peuvent être combinés avec, ou inclure, d'autres marqueurs moléculaires en vue d'augmenter encore sa sensibilité.

La présente invention permet d'améliorer la stratégie de dépistage ainsi que les traitements des formes précoces de cancer ou précancéreuses.

## Revendications

1. Procédé ou méthode permettant le pronostic ou le suivi thérapeutique du cancer colorectal ou sa mise en évidence en vue d'un diagnostic, comprenant les étapes suivantes :
a. l'extraction d'un matériel nucléique d'un échantillon biologique ;
b. la modification du matériel nucléique extrait;
c. l'utilisation d'au moins une paire d'amorces d'amplification en vue d'obtenir des amplicons d'au moins une séquence cible;
d. l'utilisation d'au moins une sonde de détection pour détecter la présence desdits amplicons ;
**caractérisé en ce que** l'échantillon biologique contient des cellules circulantes et du matériel nucléique issus d'au moins un effluent biologique, choisi au moins dans la combinaison de l'urine et du sérum, et **en ce que** ladite séquence cible est comprise dans le gène WIF-1.

2. Procédé ou méthode permettant le pronostic ou le suivi thérapeutique du cancer colorectal ou sa mise en évidence en vue d'un diagnostic, comprenant les étapes suivantes :
a. l'extraction d'un matériel nucléique d'un échantillon biologique ;
b. la modification du matériel nucléique extrait;
c. l'utilisation d'au moins une paire d'amorces d'amplification en vue d'obtenir des amplicons de la cible,
d. l'utilisation d'au moins une sonde de détection pour détecter la présence desdits amplicons ;
**caractérisé en ce que** l'échantillon biologique contient des cellules circulantes et du matériel nucléique issu du sang et **en ce que** la séquence cible est SEQ ID n°3.

3. Procédé ou méthode, selon l'une des revendications précédentes, où les étapes c) et d) sont réalisées en même temps.

4. Procédé ou méthode, selon l'une des revendications précédentes, **caractérisé en ce que** ladite étape c) est une PCR conventionnelle ou quantitative.

5. Procédé ou méthode, selon l'une des revendications précédentes, **caractérisé en ce que** ladite sonde de détection comprend un marqueur.

6. Procédé ou méthode, selon une des revendications 1, 3, 4 ou 5, **caractérisé en ce que** ladite paire d'amorces comprend au moins une amorce d'amplification comprenant au moins 15 motifs nucléotidiques d'une séquence nucléotidique choisie parmi les SEQ ID n° 1 à 10 et/ou ladite sonde de détection comprend au moins 15 motifs nucléotidiques d'une séquence nucléotidique choisie parmi SEQ ID n° 1 à 10.

7. Procédé ou méthode, selon l'une des revendications 2 à 5, **caractérisé en ce que** ladite paire d'amorces comprend au moins une amorce d'amplification comprenant au moins 15 motifs nucléotidiques d'une séquence nucléotidique choisie parmi les SEQ ID n°3, 8, 9 ou 10 et/ou ladite sonde de détection comprend au moins 15 motifs nucléotidiques d'une séquence nucléotidique choisie parmi SEQ ID n°3, 8, 9 ou 10.

8. Utilisation pour le diagnostic/pronostic et le suivi du cancer colorectal d'au moins une amorce d'amplification pour l'amplification d'une séquence cible comprise dans le gène WIF1, et/ou d'au moins une sonde de détection destinée à la détection des méthylations d'une séquence cible comprise dans le gène WIF1, **caractérisée en ce qu'**on utilise le procédé selon l'une quelconque des revendications 1 à 7.

9. Utilisation d'un kit dans le procédé ou la méthode selon l'une des revendications 1, ou 3 à 6, le kit comprenant au moins une amorce d'amplification et/ou une sonde de détection comprenant au moins 15 motifs nucléotidiques d'une séquence nucléotidique choisie parmi les SEQ ID n° 1 à 10.

10. Utilisation d'un kit dans le procédé ou la méthode selon la revendication 2 ou 7, le kit comprenant au moins une amorce d'amplification et/ou une sonde de détection comprenant au moins 15 motifs nucléotidiques d'une séquence nucléotidique choisie parmi les SEQ ID n°3, 8, 9 ou 10.

## Patentansprüche

1. Verfahren oder Methode zur Prognose oder therapeutischen Nachsorge von Darmkrebs bzw. zum Nachweis für eine Diagnose, das folgende Schritte umfasst:
a. Extraktion von Nucleinsäuren aus einer biologischen Probe;
b. Modifizieren der exktrahierten Nucleinsäuren;
c. Anwendung von mindestens einem Primerpaar zur Amplifikation, um Amplikone von mindestens einer Target-Sequenz zu erhalten;
d. Anwendung von mindestens einer Detektionssonde, um das Vorhandensein dieser Amplikone zu detektieren;
**dadurch gekennzeichnet, dass** die biologische Probe zirkulierende Zellen und Nucleinsäuren aus mindestens einer biologischen Flüssigkeit enthält, die aus der Gruppe von Urin und Serum gewählt wird, und dadurch, dass die Target-Sequenz im Gen WIF-1 enthalten ist.

2. Verfahren oder Methode zur Prognose oder therapeutischen Nachsorge von Darmkrebs bzw. zum Nachweis für eine Diagnose, das folgende Schritte umfasst:
e. Extraktion von Nucleinsäuren aus einer biologischen Probe;
f. Modifizieren der exktrahierten Nucleinsäuren;
g. Anwendung von mindestens einem Primerpaar zur Amplifikation, um Amplikone des Targets zu erhalten;
h. Anwendung von mindestens einer Detektionssonde, um das Vorhandensein dieser Amplikone zu detektieren;
**dadurch gekennzeichnet, dass** die biologische Probe zirkulierende Zellen und Nucleinsäuren aus Blut enthält, und dadurch, dass es sich bei der Target-Sequenz um die SEQ ID Nr. 3 handelt.

3. Verfahren oder Methode nach einem der vorausgehenden Ansprüche, bei dem die Schritte c) und d) gleichzeitig durchgeführt werden.

4. Verfahren oder Methode nach einem der vorausgehenden Ansprüche, **dadurch gekennzeichnet, dass** es sich beim Schritt c) um eine konventionelle oder quantative PCR handelt.

5. Verfahren oder Methode nach einem der vorausgehenden Ansprüche, **dadurch gekennzeichnet, dass** die Detektionssonde einen Marker enthält.

6. Verfahren oder Methode nach einem der Ansprüche 1, 3, 4 oder 5, **dadurch gekennzeichnet, dass** das Primerpaar mindestens einen Amplifikationsprimer mit mindestens 15 Nucleotidmustern einer unter den SEQ ID Nr. 1 bis 10 ausgewählten Nucleotidsequenz umfasst und/oder die Detektionssonde mindestens 15 Nucleotidmuster einer unter den SEQ ID Nr. 1 bis 10 ausgewählten Nucleotidsequenz umfasst.

7. Verfahren oder Methode nach einem der Ansprüche 2 bis 5, **dadurch gekennzeichnet, dass** das Primerpaar mindestens einen Amplifikationsprimer mit mindestens 15 Nucleotidmustern einer unter den SEQ ID Nr. 3, 8, 9 oder 10 ausgewählten Nucleotidsequenz umfasst und/oder die Detektionssonde mindestens 15 Nucleotidmuster einer unter den SEQ ID Nr. 3, 8, 9 oder 10 ausgewählten Nucleotidsequenz umfasst.

8. Anwendung für die Diagnose/Prognose und die Nachsorge von Darmkrebs eines Amplifikationsprimers für die Amplifikation einer im Gen WIF1 enthaltenen Target-Sequenz, und/oder mindestens einer Detektionssonde für die Detektion der Methylierungen einer im Gen WIF1 enthaltenen Target-Sequenz, **dadurch gekennzeichnet, dass** man das Verfahren nach einem der Ansprüche 1 bis 7 anwendet.

9. Anwendung eines Kits beim Verfahren oder bei der Methode nach einem der Ansprüche 1, bzw. 3 bis 6, wobei das Kit mindestens einen Amplifikationsprimer und/oder eine Detektionssonde mit mindestens 15 Nucleotidmustern einer unter den SEQ ID Nr. 1 bis 10 ausgewählten Nucleotidsequenz umfasst.

10. Anwendung eines Kits beim Verfahren oder bei der Methode nach Anspruch 2 oder 7, wobei das Kit mindestens einen Amplifikationsprimer und/oder eine Detektionssonde mit mindestens 15 Nucleotidmustern einer unter den SEQ ID Nr. 3, 8, 9 oder 10 ausgewählten Nucleotidsequenz umfasst.

## Claims

1. A process or method for the prognosis or therapeutic monitoring of colorectal cancer or its detection with a view to diagnosis, comprising the following steps:
a. extracting nucleic material from a biological sample;
b. modifying the extracted nucleic material;
c. using at least one pair of amplification primers in order to obtain amplicons of at least one target sequence;
d. using at least one detection probe to detect the presence of said amplicons;
**characterized in that** the biological sample contains circulating cells and nucleic material from at least one biological discharge, selected at least from the combination of urine and serum, and **in that** said target sequence is included in the gene WIF-1.

2. A process or method for the prognosis or therapeutic monitoring of colorectal cancer or its detection with a view to diagnosis, comprising the following steps:
a. extracting nucleic material from a biological sample;
b. modifying the extracted nucleic material;
c. using at least one pair of amplification primers in order to obtain amplicons of the target,
d. using at least one detection probe to detect the presence of said amplicons;
**characterized in that** the biological sample contains circulating cells and nucleic material from blood and **in that** the target sequence is SEQ ID no. 3.

3. A process or method according to any of the preceding claims, wherein the steps (c) and (d) are carried out simultaneously.

4. A process or method according to any of the preceding claims, **characterized in that** said step (c) is a conventional or quantitative PCR.

5. A process or method according to any of the preceding claims, **characterized in that** said detection probe comprises a marker.

6. A process or method according to any of claims 1, 3, 4 or 5, **characterized in that** said pair of primers comprises at least one amplification primer comprising at least 15 nucleotide patterns of a nucleotide sequence selected from SEQ ID no. 1 to 10 and/or said detection probe comprises at least 15 nucleotide patterns of a nucleotide sequence selected from SEQ ID no. 1 to 10.

7. A process or method according to any of claims 2 to 5, **characterized in that** said pair of primers comprises at least one amplification primer comprising at least 15 nucleotide patterns of a nucleotide sequence selected from SEQ ID no. 3, 8, 9 or 10 and/or said detection probe comprises at least 15 nucleotide patterns of a nucleotide sequence selected from SEQ ID no. 3, 8, 9 or 10.

8. Use for the diagnosis/prognosis and monitoring of colorectal cancer of at least one amplification primer for the amplification of a target sequence included in the gene WIF1, and/or at least one detection probe intended for the detection of methylations of a target sequence included in the gene WIF1, **characterized in that** the method according to any of claims 1 to 7 is used.

9. Use of a kit in the process or method according to any of claims 1 or 3 to 6, wherein said kit comprises at least one amplification primer and/or detection probe comprising at least 15 nucleotide patterns of a nucleotide sequence selected from SEQ ID no. 1 to 10.

10. Use of a kit in the process or method according to any of claims 2 or 7, wherein said kit comprises at least one amplification primer and/or detection probe comprising at least 15 nucleotide patterns of a nucleotide sequence selected from SEQ ID no. 3, 8, 9 or 10.


### SEQUENCE LISTING

<110> APHP
<120> Méthode, procédé, et kit de diagnostic, pronostic du cancer colorectal
<160> 10
<210> 1
   <211> 800
   <212> DNA
   <213> human
<400> 1
<210> 2
   <211> 267
   <212> DNA
   <213> human
<400> 2
<210> 3
   <211> 69
   <212> DNA
   <213> human
<400> 3
<210> 4
   <211> 20
   <212> DNA
   <213> human
<400> 4
   TataCGttta tttCGCGggC
   20
<210> 5
   <211> 21
   <212> DNA
   <213> human
<400> 5
   ACGACGACCC GACCTCCGCC C
   21
<210> 6
   <211> 27
   <212> DNA
   <213> human
<400> 6
   CGtatttCGt tCGCGttagt ttttttC
   27
<210> 7
   <211> 21
   <212> DNA
   <213> human
<400> 7
   CTCTCCTCCT ACGACGACCC G
   21
<210> 8
   <211> 19
   <212> DNA
   <213> human
<400> 8
   TTTGACGGCG TTAGGTTGC
   19
<210> 9
   <211> 24
   <212> DNA
   <213> human
<400> 9
   ACTACTCAAA ACCTCCTCGC TACC
   24
<210> 10
   <211> 16
   <212> DNA
   <213> human
<400> 10